# EUROPEAN PATENT APPLICATION

(11) **EP 1 695 618 A2**
(43) Date of publication of application: **30.08.2006**
(21) Application number: 06003087.1
(22) Date of filing: 15.02.2006
(51) Int. Cl.: A01K 67/02, A01K 5/02

(54) **Method of increasing the fertility of female animals**

(30) Priority: 24.02.2005 DK 200500281
(71) Applicant: TWINCA A/S, 7830 Vinderup (DK)
(72) Inventor: Strom Kristensen, Mads, 7830 Vinderup (DK); Strom Kristensen,Klaus, 7830 Vinderup (DK)
(74) Representative: Schmidt, Jens Joergen

(57) **Abstract**

A method for increasing the fertility of female furred animals such as mink is disclosed, where the fertility is increased by manipulation of the quantity of feed distributed to the individual animal during the mating season, meaning that each female will bear even more cubs per litter, when the female is exposed to a second flush prior to a second mating within the same mating season.

The method comprising the steps of daily individual feeding of each of the animals by a predetermined quantity of feed for a first period of time, daily individual feeding of each of the animals by a substantially larger quantity of feed for a second period of time, first mating each of the female animals, determining by means of the control system a starting date of a fourth period of time for each of said animals from recorded dates of the first mating, daily individual feeding of each of the animals by a smaller quantity of feed for a third period of time, daily individual feeding of each of the animals by a larger quantity of feed for the fourth period of time, and second mating each of the female animals at the end of the fourth period of time.

## Description

The present invention relates to a method for increasing the fertility of female furred animals such as mink, where the fertility is increased by manipulation of the quantity of feed distributed to the individual animal during the mating season.

### BACKGROUND

Flush is a known method for increasing the fertility of caged female animals primarily mink and other furred animals. With this method the normal quantity of feed distributed to the female animal is manipulated for a period of time when the female is in heat, and the female is normally mated twice. The high amount of accessible feed will stimulate the ovulation whereby she will bear more cubs. This form of flush can be controlled by ordinary automatic feeding equipment which is used today or to a certain extend by manual feeding.

It is an object of the present invention to further increase the number of cubs per litter for the individual female animal.

### BRIEF DESCRIPTION OF THE PRESENT INVENTION

It has been realized by the present inventor that the fertility of the female can be increased further, meaning that each female will bear even more cubs per litter, if the female is exposed to a second flush prior to a second mating within the same mating season.

Thus, the present invention relates to a method for increasing the fertility of each of a plurality of female animals by controlled feeding, the method comprising the steps of daily individual feeding of each of the animals by a predetermined quantity of feed for a first period of time, daily individual feeding of each of the animals by a predetermined quantity of feed for a second period of time following the first period of time, wherein the predetermined quantity of feed in the second period of time is substantially larger for each of the animals than in the first period of time, first mating each of the female animals and entering data of the date of the first mating for each individual of the animals into a control system where a record is kept for each individual animal, determining by means of the control system a starting date of a fourth period of time for each of said animals from said recorded dates of the first mating, daily individual feeding of each of the animals by a predetermined quantity of feed for a third period of time following the mating, wherein the predetermined quantity of feed in the third period of time is substantially smaller for each of the animals than in the second period of time, daily individual feeding of each of the animals by a predetermined quantity of feed for the fourth period of time following the third period of time, wherein the predetermined quantity of feed in the fourth period of time is substantially larger for each of the animals than in the third period of time, and second mating each of the female animals at the end of the fourth period of time.

By the term substantially is meant, that the predetermined quantity of feed for the individual animal is decreased or increased with a large percentage in relation to the normal or the previous quantity of feed distributed to the animal, e.g. that the quantity of feed is doubled or halved. The time periods, where the manipulation of feed takes place, are within a plurality of days.

This method is preferably employed within the same mating season for seasonal breeders, that is animals where all the females are in heat at the same time.

The control system employed may further control an apparatus for automatically metering out the predetermined quantity of feed for each of the animals.

In an embodiment of this method, the predetermined quantity of feed in the first period of time is reduced for each of the animals as compared to the normal quantity of feed fed to the animals, preferably with at least 25% of the normal quantity of feed.

The said first period of time might be within 3 to 14 days of length, preferably within 4 to 9 days of length.

The predetermined quantity of feed in the second period of time may be at least 100% higher than the predetermined quantity of feed in the first period of time, preferably at least 200% higher.

The said second period of time might be within 2 to 12 days of length, preferably within 3 to 8 days of length.

It is preferred that the first mating of the female ends the second period and starts the third period.

Hereafter the predetermined quantity of feed in the third period of time might be reduced with at least 40% as compared to the quantity of feed in the second period, preferably with at least 60%.

The number of days from the date of the first mating and the starting date of the fourth period of time for each of said animal might be within 4 to 10 days.

The predetermined quantity of feed in the fourth period of time may at least be 80% higher than the predetermined quantity of feed in the third period of time, preferably at least 140% higher.

It is preferred that the second mating of the female takes place 2 to 5 days after the beginning of the fourth period of time.

### BRIEF DESCRIPTION OF THE DRAWING

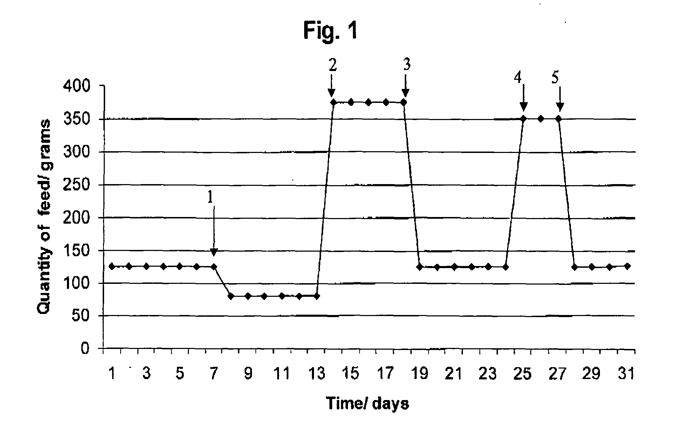

An embodiment of the present invention is shown on the graph in fig. 1. The graph is a timeline of the quantity of feed distributed to an animal in accordance with an embodiment of the invented method.

### DETAILED DESCRIPTION OF EMBODIMENTS OF THE PRESENT INVENTION

To be efficient, it is advantageous that the method is performed by means of a system that can handle multiple information at the same time. The system has to keep track on the first mating date of each female, and preferably also which cage the female is placed in, the animal ID, the date where the quantity of feed is set normal and which percentage the normal quantity of feed has to be increased or decreased during first and second flush. The system would preferably also store information from previously mating of the female animal in order to optimize the consumption of feed.

With the term manipulation of feed is understood that the normal quantity of feed is decreased or increased in a period from just before and during the mating season.

A portable registration unit is used to keep track of which cage the individual animal is placed in, its ability to consume feed and its state of health. This insures that the individual animal is feed correctly. The portable unit could be a hand held computer, e.g. a PDA. For easy registration of the cages, the portable unit could also be equipped with a barcode scanner, and the individual cage with a unique barcode. This portable unit could also serve as a link between the apparatus which controls the manipulation of feed and the system which distributes the feed to the animals.

It is preferred that the apparatus for controlling the manipulation of feed consists of a standard computer equipped with a program to execute the calculations of the quantity of feed for the individual animal and the length of the time periods where the quantity of feed is decreased or increased.

The manipulation of the feed quantity takes place just before and during the mating season. With the term manipulation is understood that the normal quantity of feed for the female is lowered for some days, where after the quantity of feed is increased considerably over the normal level for a short period. By the end of this period, with a high quantity of feed, the female is mated, where after the quantity of feed is lowered back to the normal level. After some days at the normal level, the quantity of feed is raised again for some days, and the female is mated a second time. Hereafter the quantity of feed is returned to the normal level.

An example of such manipulation is shown on the graph in fig. 1, where the quantity of feed for one female is shown as a function of time. The normal quantity of feed for this individual female is 125g of feed per day. When the first period starts (1), the quantity of feed is reduced to 80g of feed per day over duration of 6 days. Hereafter the second period starts (2), and the female is exposed to the first flush at which the quantity of feed distributed to the female is increased to 375g per day over duration of 5 days. At the end of the second period (3) the female is mated for the first time, where after the quantity of feed is decreased back to 125g per day. Here the third period starts and has a duration of 6 days, where after the fourth period starts (4). In this period, which has duration of 3 days, the quantity of feed is increased again, this time to 350g per day, whereby the female is exposed for the second flush. At the end (5) of the fourth period the female is mated for the second time, where after the quantity of feed for the female is decreased back to the normal 125g per day.

The system for distributing the feed to the animals could be a feed cart which is designed to enable the operator, by means of a discharge hose, to dose out rather accurate paste-like wet feed portions on the net top of the cages. The size of the feed portion for each cage is then controlled by the portable unit on background of information from the apparatus which calculates the manipulation of the quantity of feed.

In another embodiment of this invention, the system for distributing the feed could also consist of automatically operated dispensers on each cage controlled directly by the apparatus which calculates the manipulation of the quantity of the feed.

Again the preferred apparatus for the control of the feed would be a standard computer. Instead of wet feed this system could distribute the feed in form of dry fodder via the automatically operated dispensers. The advantage of this method would be savings in labour when feeding the animals, as the dispensers only have to be filled up at intervals of a few days.

The automatically operated dispensers could also be supplied from a central silo via a system of pipes or conveyor belts, and the feed could be in form of both paste and dry fodder. This more effective method, for distributing the feed, will further save costs in labour for the feeding of the animals.

## Claims

1. Method for increasing the fertility of each of a plurality of female animals by controlled feeding, the method comprising the steps of
daily individual feeding of each of the animals by a predetermined quantity of feed for a first period of time,
daily individual feeding of each of the animals by a predetermined quantity of feed for a second period of time following the first period of time, wherein the predetermined quantity of feed in the second period of time is substantially larger for each of the animals than in the first period of time,
first mating each of the female animals and entering data of the date of the first mating for each individual of the animals into a control system where a record is kept for each individual animal,
determining by means of the control system a starting date of a fourth period of time for each of said animals from said recorded dates of the first mating,
daily individual feeding of each of the animals by a predetermined quantity of feed for a third period of time following the mating, wherein the predetermined quantity of feed in the third period of time is substantially smaller for each of the animals than in the second period of time,
daily individual feeding of each of the animals by a predetermined quantity of feed for the fourth period of time following the third period of time, wherein the predetermined quantity of feed in the fourth period of time is substantially larger for each of the animals than in the third period of time, and
second mating each of the female animals at the end of the fourth period of time.

2. Method according to claim 1, wherein the control system controls an apparatus for automatically metering out the predetermined quantity of feed for each of the animals.

3. Method according to claim 1 or 2, wherein the predetermined quantity of feed in the first period of time is reduced for each of the animals as compared to the normal quantity of feed fed to the animals.

4. Method according to claim 3, wherein said predetermined amount of feed is reduced with at least 25% of the normal quantity of feed.

5. Method according to claim 3 or 4, wherein said first period of time is within 3 to 14 days of length, preferably within 4 to 9 days of length.

6. Method according to any of the preceding claims, wherein the predetermined quantity of feed in the second period of time is at least 100% higher than the predetermined quantity of feed in the first period of time, preferably at least 200% higher.

7. Method according to any of the preceding claims, wherein said second period of time is within 2 to 12 days of length, preferably within 3 to 8 days of length.

8. Method according to any of the preceding claims, wherein the first mating takes place at the end of the second period of time.

9. Method according to any of the preceding claims, wherein the predetermined quantity of feed in the third period of time is reduced with at least 40% as compared to the quantity of feed in the second period, preferably with at least 60%.

10. Method according to any of the preceding claims, wherein the number of days from the date of the first mating and the starting date of the fourth period of time for each of said animals is within 4 to 10 days.

11. Method according to any of the preceding claims, wherein the predetermined quantity of feed in the fourth period of time is at least 80% higher than the predetermined quantity of feed in the third period of time, preferably at least 140% higher.

12. Method according to any of the preceding claims, wherein the second mating takes place 2 to 5 days after the beginning of the fourth period of time.

13. An apparatus for automated individual feeding of animals, having a control system adapted to control the feeding of a plurality of female animals in accordance with the method of any of claims 1-12.
